# EUROPEAN PATENT APPLICATION

(11) **EP 3 345 641 A1**
(43) Date of publication of application: **11.07.2018**
(21) Application number: 17150593.6
(22) Date of filing: 06.01.2017
(51) Int. Cl.: A61M 5/32, A61M 25/06

(54) **SAFETY SYRINGES**

(71) Applicant: Anhui Hongyu Wuzhou Medical Manufacturer Co., Ltd, Beijing 102200 (CN); Wang, Hsien Tsung, Taipei 10061 (TW)
(72) Inventor: Wang, Hsien Tsung, Taipei 10061 (TW)
(74) Representative: Baldwin, Mark

(57) **Abstract**

A safety syringe in one embodiment includes an attachment seat including a channel and two wings; a hypodermic needle; a lower clamping member below the attachment seat; an upper clamping member on the attachment seat and releasably secured to the lower clamping member; and a needle sheath put on the attachment seat and configured to slide in both the lower and upper clamping members. The upper clamping member includes a U-shaped opening on an outer surface. The needle sheath includes a rear projection on an outer surface. The needle sheath may move forward to move the rear projection until the rear projection falls into the U-shaped opening and the hypodermic needle is locked and concealed by the needle sheath.

## Description

### BACKGROUND TO THE INVENTION

### 1. Field of the Invention

The invention relates to medical syringes and more particularly to a safety syringe having a tamper-proof characteristic.

### 2. Description of Related Art

Conventionally, in a medical injection a medical employee may remove a needle sheath, temporarily secure two wings of the safety syringe to the skin of a patient to be injected, insert a needle of a safety syringe into the skin to inject medicine thereinto, and pull the needle out after the injection. The employee may put the used needle into the needle sheath prior to destroying the safety syringe. This is because the needle may be contaminated.

Safety syringes are typically used in IV (intravenous) injection. An IV injection may take several hours. A medical employee may frequently remove the IV needle during an IV injection. Also, the employee may put the used needle into the needle sheath because the needle may be contaminated. However, the employee may be accidentally pricked by the used needle if insufficient care is taken. Further, the employee who inserts the IV needle into the skin of a patient may not be the employee who removes the needle. Further, the employee removing the needle may be unable to find the removed needle sheath.

Thus, the need for improvement still exists.

### SUMMARY OF THE INVENTION

The invention provides a safety syringe comprising an attachment seat including a channel and two wings at two sides of the channel respectively;
a hypodermic needle having a rear end attached to a rear tube; a lower clamping member disposed below both the attachment seat and the rear tube; an upper clamping member disposed on both the attachment seat and the rear tube and releasably secured to the lower clamping member; and a needle sheath put on the attachment seat and configured to slide in both the lower clamping member and the upper clamping member; wherein the upper clamping member includes a U-shaped opening on an outer surface; wherein the needle sheath includes a rear projection on an outer surface; and wherein the needle sheath is configured to move forward to move the projection until the projection falls into the U-shaped opening and the hypodermic needle is locked and concealed by the needle sheath.

According to an embodiment, the attachment seat includes a channel, two wings at two sides of the channel respectively, and a stepped-diameter mount on a rear end of the hypodermic needle, the stepped-diameter mount is inserted through the channel and the rear tube to fasten them together.

According to an embodiment, the upper clamping member further comprises a limit member in the U-shaped opening, the limit member having a vertical forward surface and an inclined surface facing rearward.

According to an embodiment, the lower clamping member includes two opposite snapping members on two sides of a front end respectively, and the upper clamping member includes two opposite snapping elements on a bottom of a front end respectively, and the snapping members and the snapping elements are complimentarily engaged.

According to an embodiment, the lower clamping member includes an elongated flat bottom adjacent to a front end, two opposite engaging members on two sides of the bottom and spaced from the front end, two opposite lower grooves on inner surfaces of the engaging members respectively, and two opposite positioning members on outer surfaces of the engaging members respectively, each positioning member having an elongated slot.

According to an embodiment, the lower clamping member includes an elongated flat bottom adjacent to a front end, two opposite engaging members on two sides of the bottom and spaced from the front end, two opposite positioning members on outer surfaces of the engaging members respectively, each positioning member having an elongated slot.

According to an embodiment, the upper clamping member further comprises a front recess, a rear positioning element, two opposite cavities on bottoms of two sides of intermediate and rear portions respectively, and two opposite upper grooves on two sides of an inner surface of the rear positioning element respectively, and wherein the rear positioning element includes two elongated latches on two sides respectively, the latches are inserted through the slots to secure the upper clamping member and the lower clamping member together.

According to an embodiment, the rear projection has an inclined surface facing forward and a vertical rear surface.

According to an embodiment, the rear positioning element further comprises a gripping aiding member on an outer surface.

According to an embodiment, the lower clamping member includes two upward gripping aiding members on an outer surface.

According to an embodiment, the needle sheath further comprises two opposite elongated slots on an outer surface.

According to an embodiment, further comprising a blood collection tube connected to the safety syringe.

Embodiments of the invention may provide the following characteristics and advantages in comparison with the prior art: after an injection, a medical employee may push the trigger forward to cause the needle sheath to lockingly enclose the needle. Thus, a second use of the contaminated needle is made impossible. This is because the needle sheath is prohibited from returning to its original position between the upper clamping member and the lower clamping member by the provisions of the projection (or the like) and the front and rear limit members in a complementary manner. The slide is configured to move along the upper and lower grooves during the needle enclosing operation after the injection. The medical employee is prevented from being accidentally pricked by the used needle.

The above and other objects, features and advantages of the invention will become apparent from the following detailed description taken with the accompanying drawings.

### BRIEF DESCRIPTION OF THE DRAWINGS

FIG. 1 is a perspective view of a safety syringe according to a first preferred embodiment of the invention;
FIG. 2 is an exploded view of the safety syringe;
FIG. 2A is a perspective view of the underside of the upper clamping member;
FIG. 3 is a view similar to FIG. 1 showing the needle covered by a needle sheath;
FIG. 4 is a view similar to FIG. 1 showing the attachment seat of FIG. 1 replaced by a second attachment seat in another configuration;
FIG. 5 is an exploded view of the safety syringe of FIG. 4;
FIG. 6 is a view similar to FIG. 4 showing the needle covered by a needle sheath;
FIG. 7 is a perspective view of safety syringe of FIG. 1 showing its connection to a blood collection tube;
FIG. 8 is a perspective view of a safety syringe according to a second preferred embodiment of the invention;
FIG. 9 is an exploded view of the safety syringe of FIG. 8;
FIG. 9A is a perspective view of the underside of the upper clamping member of FIG. 9;
FIG. 10 is a view similar to FIG. 8 showing the needle covered by a needle sheath;
FIG. 11 is a view similar to FIG. 8 showing the attachment seat of FIG. 8 replaced by a second attachment seat in another configuration; and
FIG. 12 is a perspective view of safety syringe of FIG. 8 showing its connection to a blood collection tube.

### DETAILED DESCRIPTION

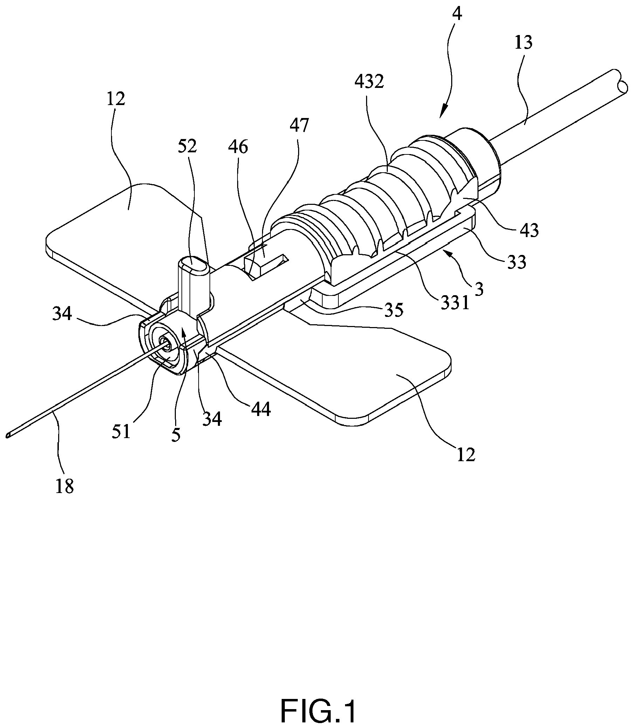

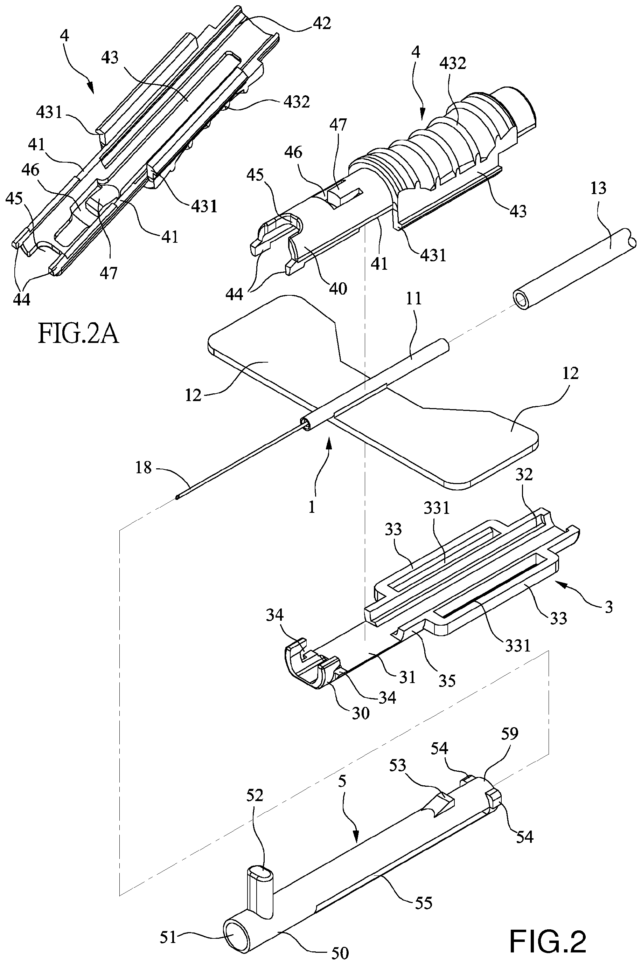

Referring to FIGS. 1, 2, 2A and 3, a safety syringe in accordance with a first embodiment of the invention comprises the components discussed in detail below.

An attachment seat 1 includes a channel, or tubular body, 11 and two wings 12 at respective sides of the channel 11. A hypodermic needle 18 has a rear portion fastened in the channel 11. A tube 13 is secured onto a rear portion of the channel 11 so as to establish a fluid path from the tube 13 to the needle 18 via the channel 11.

A lower clamping member 3 includes an elongate flat bottom surface 31 adjacent to a U-shaped front end 30, two oppositely disposed engaging members 35 on respective sides of the bottom surface 31 and spaced from the front end 30, two oppositely disposed lower grooves 32 on respective inner surfaces of the engaging members 35, two oppositely disposed positioning members 33 on respective outer surfaces of the engaging members 35, each positioning member 33 having an elongate slot 331, and two oppositely disposed snapping members 34 on respective sides of the front end 30.

An upper clamping member 4 includes two oppositely disposed snapping elements 44 at the bottom of a front end 40, a recess 45 between the snapping elements 44, a rear positioning element 43 having respective elongate latches 431 on two sides, and a grip aiding member (e.g., knurls) 432 formed on an outer surface, two oppositely disposed cavities, or recesses, 41 at the bottom of respective sides of intermediate and rear portions, two oppositely disposed upper grooves 42 on respective sides of an inner surface of the rear positioning element 43, a U-shaped opening 46 on the intermediate portion, and a limit member 47 in the U-shaped opening 46. The limit member 47 has a vertical forward surface and an inclined surface facing rearward.

A needle sheath 5 includes an axial tunnel 51, two oppositely disposed elongate slots 55 on an outer surface, an upwardly projecting trigger 52 formed on the outer surface of a front end 50 and an upwardly projecting projection 53 formed on the outer surface of a rear end 59, the projection 53 being aligned with the trigger 52 and having an inclined forward facing surface and a vertical rear facing surface, and two oppositely disposed slides, or ears, 54 formed on the outer surface of the rear end 59.

In an assembled state, the needle 18 is inserted in the tunnel 51 with the wings 12 passing through the slots 55 until being stopped. The needle 18 is exposed. The latches 431 are inserted through the slots 331 to secure the upper clamping member 4 and the lower clamping member 3 together. The trigger 52 projects out of the recess 45 and urges against the recess 45. The snapping members 34 on the first clamping member 3 and the snapping elements 44 on the second clamping member 4 are complementarily engaged. The slides 54 of the needle sheath 5 are disposed at respective rear ends of the upper grooves 42 and the lower grooves 32 and configured to slide forwardly along the grooves 42, 32. The projection 53 is stopped by a rear end of a recessed portion on an inner surface of the positioning element 43 in an inoperative default position. The needle 18, the attachment seat 1, and the needle sheath 5 are fastened together by both the lower clamping member 3 and the upper clamping member 4.

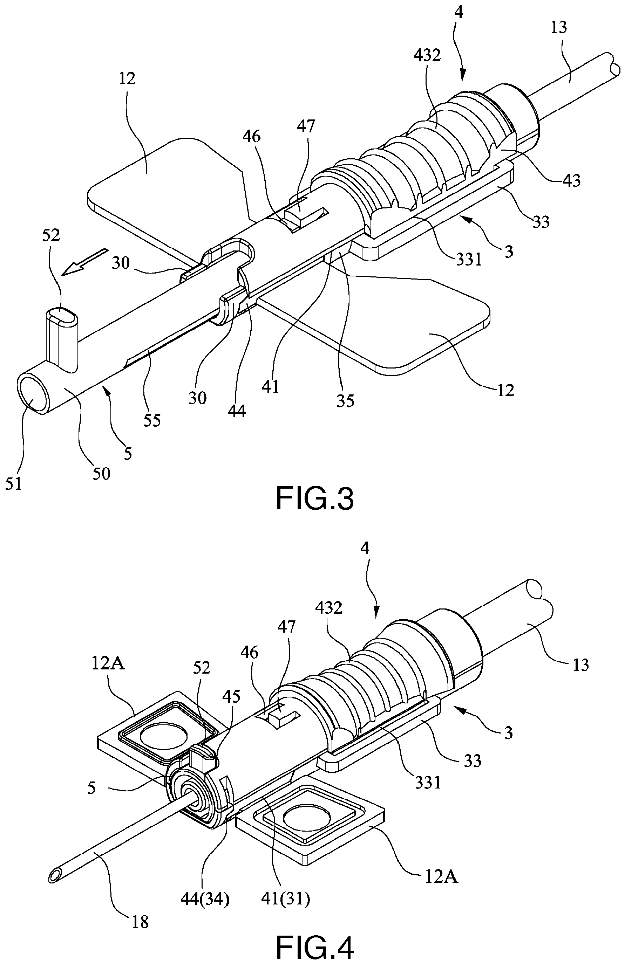

As shown in FIG. 3, after an injection a medical employee may push the trigger 52 forward to move the projection 53 with the slides 54 along the upper and lower grooves 42, 32 until the projection 53 passes the limit member 47 and falls, or snaps, into the U-shaped opening 46 to be locked therein. At this position, the needle 18 is locked and concealed by the needle sheath 5. Thus, a second use of the contaminated needle 18 is made impossible.

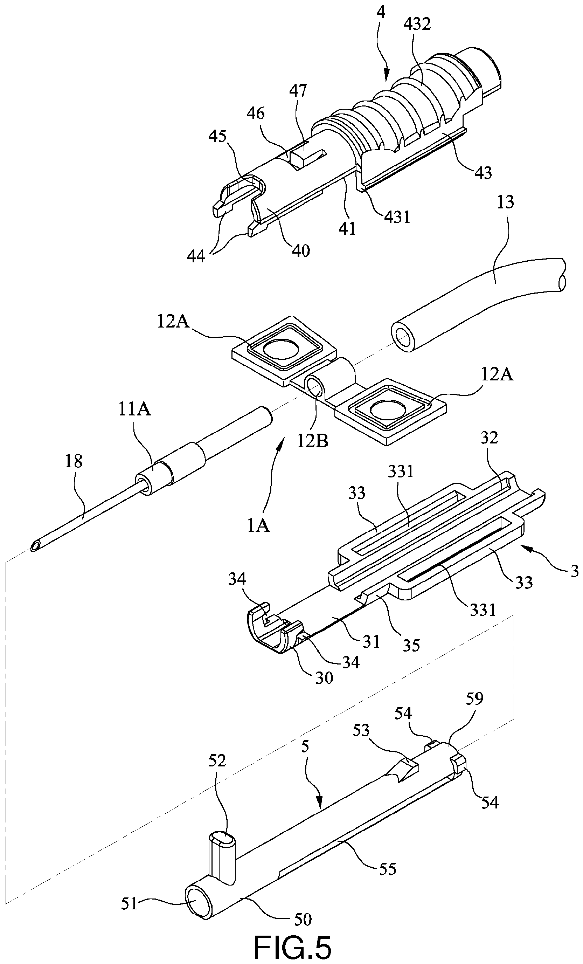

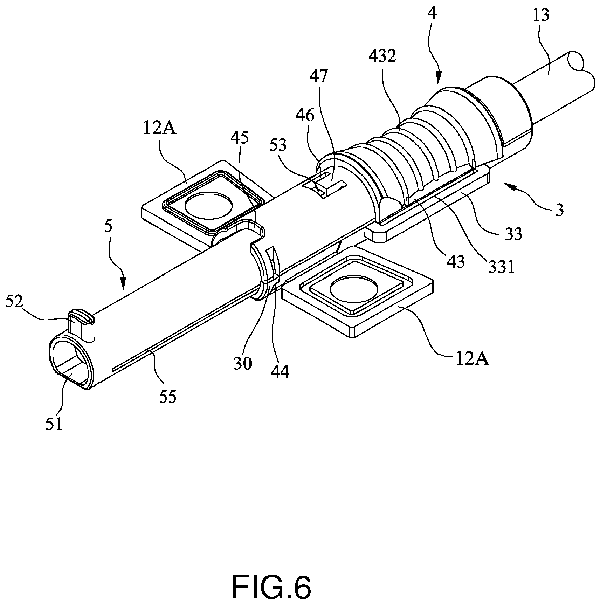

Referring to FIGS. 4, 5 and 6, another configuration of the first preferred embodiment is shown. The difference between this configuration and the configuration shown in Figures 1 to 3 is that the attachment seat 1A includes two wings 12A different from the wings 12, a channel 12B formed with two wings 12A, and a stepped-diameter channel 11 A formed on a rear end of the needle 18 that is inserted through the channel 12B and the tube 13 to fasten them together.

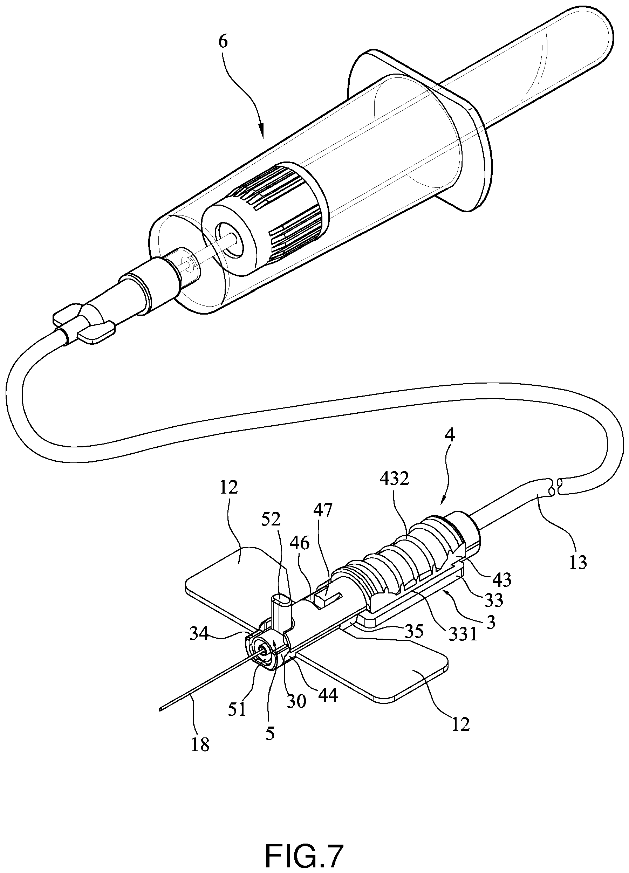

FIG. 7 shows the safety syringe shown in FIGS. 1 to 3 connected to a blood collection tube 6 for a blood drawing operation.

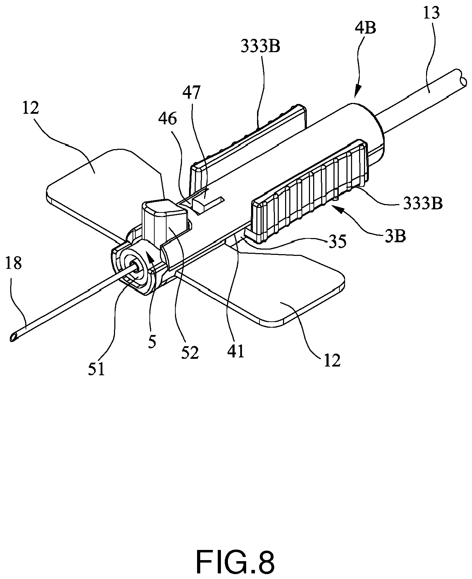

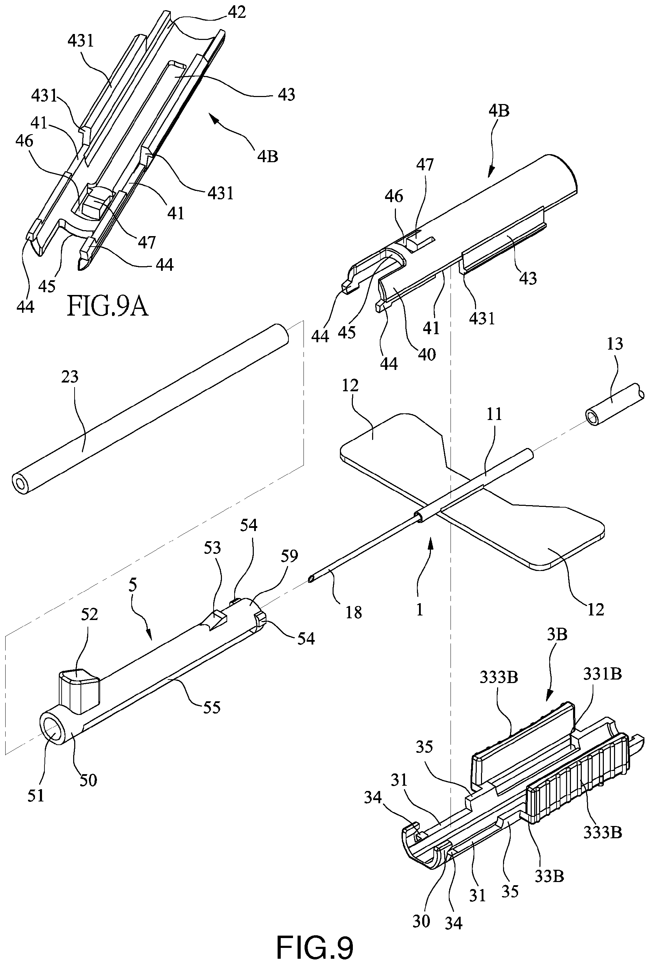

Referring to FIGS. 8, 9, 9A and 10, a safety syringe in accordance with a second embodiment of the invention is shown. The characteristics of the second embodiment are substantially the same as that of the first embodiment. A needle protection tube 23 is placed on the hypodermic needle 18 and secured to a front portion of the channel 11. A lower clamping member 3B includes an elongate flat bottom 31 adjacent to a U-shaped front end 30, two oppositely disposed engaging members 35 on respective sides of the bottom surface 31 and spaced from the front end 30, two oppositely disposed positioning members 33B on respective outer surfaces of the engaging members 35, each positioning member 33B having an elongate slot 331 B, and two oppositely disposed snapping members 34 on opposite sides of the front end 30 respectively. A rectangular grip aiding member (e.g., knurls) 333B is formed on the positioning member 33B and oriented upward.

An upper clamping member 4B includes two oppositely disposed snapping elements 44 on the bottom of a front end 40, a recess 45 between the snapping elements 44, a rear positioning element 43 having two elongate latches 431 on respective sides, two oppositely disposed cavities 41 on the bottom of respective sides of intermediate and rear portions respectively, two oppositely disposed upper grooves 42 on respective sides of an inner surface of the rear positioning element 43, a U-shaped opening 46 on the intermediate portion, and a limit member 47 in the U-shaped opening 46. The limit member 47 has a vertical forward facing surface and an inclined rearward facing surface. Each latch 431 is fastened at a joining portion of the grip aiding member 333B and the positioning member 33B.

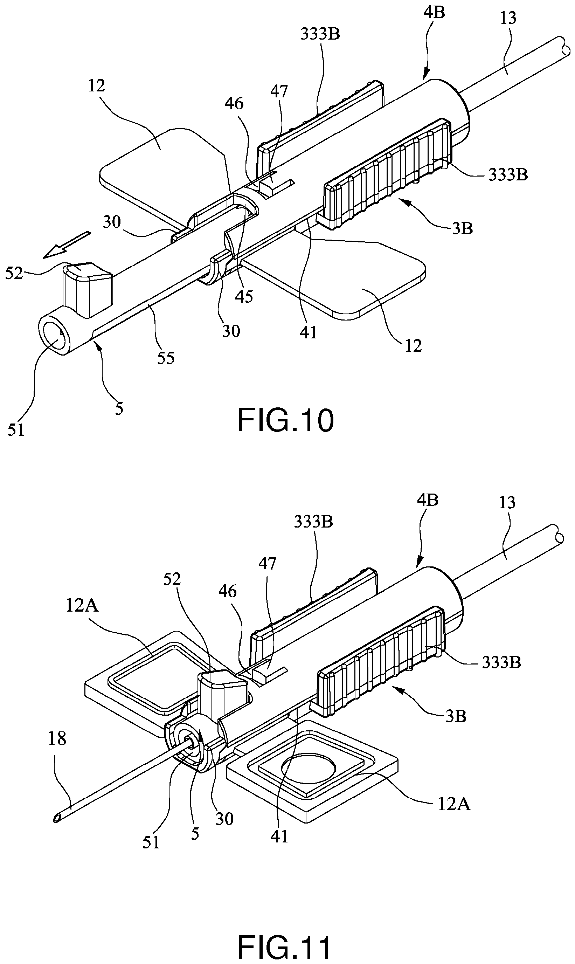

Referring to FIG. 11, another configuration of the second embodiment is shown. The difference between this configuration and the configuration shown in Figures 8 to 10 is that the attachment seat includes two wings 12A different from the wings 12.

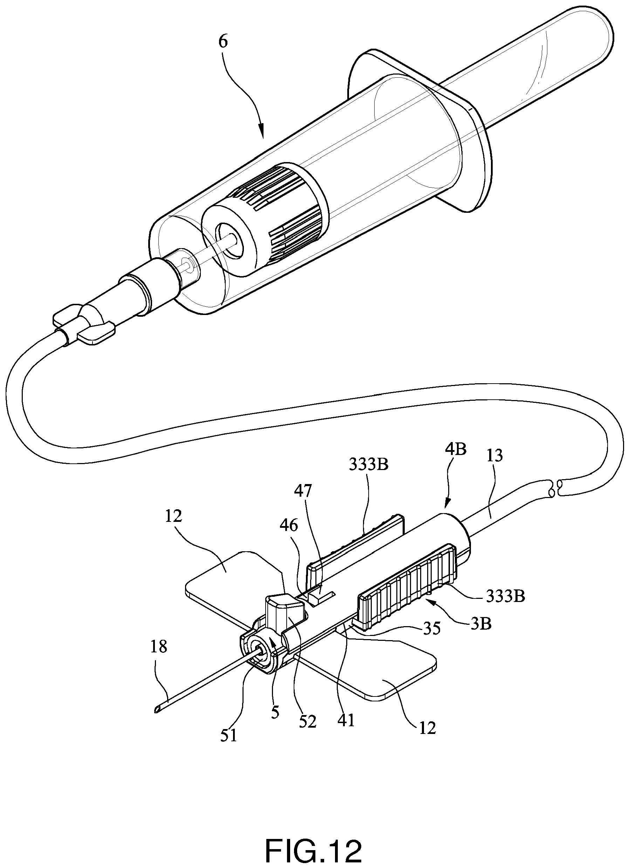

FIG. 12 shows the safety syringe shown in FIGS. 8, 9, 9A and 10 connected to a blood collection tube 6 for a blood drawing operation.

The embodiments have the following characteristics and advantages: after an injection, a medical employee may push the trigger forward to cause the needle sheath to lockingly enclose the needle. Thus, a second use of the contaminated needle is made impossible. This is because the needle sheath is prohibited from returning to its original position between the upper clamping member and the lower clamping member by the provision of the projection (or the like) and the front and rear limit members in a complementary manner. The needle sheath is configured to slide forward guided by upper and lower grooves during the needle enclosing operation after the injection. The medical employee is prevented from being accidentally pricked by the used needle.

While the invention has been described in terms of illustrated embodiments, those skilled in the art will recognise that the invention can be practiced with modifications within the scope of the appended claims.

## Claims

1. A safety syringe comprising:
an attachment seat (1, 1 A) including a channel (11, 12B) and respective wings (12, 12A) at two sides of the channel (11, 12B);
a hypodermic needle (18) having a rear end attached to a rear tube (13);
a lower clamping member (3, 3B) disposed below both the attachment seat (1, 1A) and the rear tube (13);
an upper clamping member (4, 4B) disposed on both the attachment seat (1, 1 A) and the rear tube (13) and releasably secured to the lower clamping member (3, 3B); and
a needle sheath (5) on the attachment seat (1, 1A) and configured to slide in both the lower clamping member (3, 3B) and the upper clamping member (4, 4B),
wherein the upper clamping member (4, 4B) includes an opening (46), an outer surface of the needle sheath (5) is provided with a rear projection (53) and the needle sheath (5) is configured to move forward to move the rear projection (53) until the rear projection (53) is received in the opening (46) and the hypodermic needle (18) is locked and concealed by the needle sheath (5).

2. The safety syringe of claim 1, wherein rear end of the hypodermic needle (18) is provided with a stepped-diameter channel (11A) that is inserted in the channel (12B) and the rear tube (13) to fasten them together.

3. The safety syringe of claim 1 or 2, wherein the upper clamping member (4, 4B) further comprises a limit member (47) having a vertical forward facing surface and an inclined rearward facing surface and said opening (46) is a U-shaped opening partially defined by said limit member.

4. The safety syringe of claim 1, 2 or 3, wherein the lower clamping member (3, 3B) includes two oppositely disposed snapping members (34) on respective sides of a front end (30) and the upper clamping member (4, 4B) includes two oppositely snapping elements (44) on the bottom of a front end (40), and the snapping members (34) and the snapping elements (44) are complementarily engaged.

5. The safety syringe of any one of the preceding claims, wherein the lower clamping member (3) includes an elongate flat bottom surface (31) adjacent to a front end (30), two oppositely disposed engaging members (35) on respective sides of the bottom surface (31) and spaced from the front end (30), two oppositely disposed lower grooves (32) on respective inner surfaces of the engaging members (35), and two oppositely disposed positioning members (33) on respective outer surfaces of the engaging members (35), each positioning member (33) having an elongate slot (331).

6. The safety syringe of claim 5, wherein the upper clamping member (4) further comprises a front recess (45), a rear positioning element (43), two oppositely disposed cavities (41) at the bottom of respective sides of intermediate and rear portions, and two oppositely disposed upper grooves (42) on respective sides of an inner surface of the rear positioning element (43), and wherein the rear positioning element (43) includes two elongate latches (431) on respective sides and the latches (431) are inserted through the slots (331) to secure the upper clamping member (4) and the lower clamping member (3) together.

7. The safety syringe of any one of claims 1 to 4, wherein the lower clamping member (3B) includes an elongate flat bottom surface (31) adjacent to a front end (30), two oppositely disposed engaging members (35) on respective sides of the bottom surface (31) and spaced from the front end (30) and two oppositely disposed positioning members (33B) on respective outer surfaces of the engaging members (35), each positioning member (33B) having an elongate slot (331 B).

8. The safety syringe of claim 7, wherein the upper clamping member (4B) further comprises a front recess (45), a rear positioning element (43), two oppositely disposed cavities (41) at the bottom of respective sides of intermediate and rear portions, and two oppositely disposed upper grooves (42) on respective sides of an inner surface of the rear positioning element (43), and wherein the rear positioning element (43) includes two elongate latches (431) on respective sides and the latches (431) are inserted through the slots (331 B) to secure the upper clamping member (4) and the lower clamping member (3B) together.

9. The safety syringe of claim 7 or 8, wherein the rear positioning element (43) further comprises a grip aiding member (432) on an outer surface.

10. The safety syringe of claim 7, 8 or 8, wherein the lower clamping member (3B) includes two upwardly extending grip aiding members (333B) on an outer surface.

11. The safety syringe of any one of the preceding claims, wherein the rear projection (53) has an inclined forward facing surface and a vertical rear facing surface.

12. The safety syringe of any one of the preceding claims, wherein the needle sheath (5) further comprises two oppositely disposed elongate slots (55) on an outer surface.

13. The safety syringe of any one of the claims, further comprising a blood collection tube (6) connected to the safety syringe.
